# EUROPEAN PATENT APPLICATION

(11) **EP 3 660 054 A1**
(43) Date of publication of application: **03.06.2020**
(21) Application number: 18838800.3
(22) Date of filing: 25.07.2018
(51) Int. Cl.: C08B 1/08, C08L 3/02, A61K 8/73, A61K 8/9794, A61Q 19/00

(54) **PROCESS FOR BLEACHING BABASSU**

(30) Priority: 28.07.2017 BR 102017016264
(71) Applicant: Natura Cosméticos S.A., 05106-000 São Paulo - SP (BR)
(72) Inventor: CANALLI ORTIZ MOREIRA, Giovanna, 05106-000 São Paulo - SP (BR); BEZERRA CLAUDIO DE AVILA, Thais, 05106-000 São Paulo - SP (BR); ARAUJO RODRIGUES MUCHIUTTI, Pâmela, 05106-000 São Paulo - SP (BR); FERNANDA DE SOUZA FERREIRA GARCIA, Cinthia, 05106-000 São Paulo - SP (BR)
(74) Representative: Simmons & Simmons
(86) International application number: PCT/BR2018/050255
(87) International publication number: WO 2019/018916

(57) **Abstract**

The present invention relates to a novel process of bleaching babassu, preferably from species *Orbignya phalerata.* The process of the present invention is performed under controlled temperature and an antifoam is added in addition to using only water in its extraction, thus providing a more efficient and cost effective process.

## Description

### Field of the Invention

The present invention relates to a novel process of bleaching babassu, preferably from species *Orbignya phalerata.*

### Background of the Invention

Several species of babassu exist, including those from species *Orbignya phalerata,* the mesocarp of which is extracted and then transformed into babassu flour.

Babassu coconut has three layers, an outer fibrous layer (epicarp); an intermediate, fibrous, starchy layer (mesocarp); and an inner woody layer (endocarp) where the almonds are present. The shell comprises the group of three layers, ie., epicarp, mesocarp and endocarp, corresponding to approximately 93% of the total coconut, and weighing up to 300 grams. The mesocarp usually represents approximately 20% of the total weight.

Fresh mesocarp color resembles light cream and it can easily be reduced to powder. As it gets older, it gets woody stiffness and a reddish-brown color. When dried, upon being soaked in water, it has a latex-like texture being hardly ground or crushed.

Thus, the natural color of babassu flour varies from beige to brown, which restricts its use in cosmetic applications such as moisturizers, lotions and other applications. In cosmetic formulations in general, the flour color migrates to the final product, making standardization difficult and reducing its possible uses.

Some babassu bleaching processes have already been described. Japanese patent document number JPS62192308 relates to a cosmetic composition comprising purified babassu oil. While mentioning the possibility of oxidative bleaching, this document is intended is to purify and use the oil.

Therefore, there remains a need for an efficient and inexpensive process for bleaching babassu flour, more preferably from *Orbignya phalerata* mesocarp extraction.

### Description of the invention

The object of the present invention is a process for bleaching babassu mesocarp, preferably *Orbignya phalerata* babassu, which is extracted and processed into flour, comprising the following steps:
- sieving the milled mesocarp, particularly through a 200 to 400 mesh sieve, to remove fibers;
- aqueous extraction, particularly at substrate:water ratios of from about 1:5 to about 1:20;
- adding hydrogen peroxide, particularly about 120 volumes;
- adding sodium hydroxide, particularly at concentrations of from about 10% to about 30% to reach a pH of about 11 to 12. Sodium hydroxide addition can be adapted to various plant sources, if required.
- adding from about 100 to about 200 mL of antifoam agent, for example polydimethylsiloxane-rich antifoam,
- filterinh, preferably using a filter press;
- washing with dilute acid, particularly hydrochloric acid and water;
- drying;
- optionally micronizing.

The use of basic hydrogen peroxide has been found to generate a large amount of foam in an exothermic reaction. Increased temperature causes the substrate, which has gelling characteristics, to form starch gel.

Thus, the process must also have its temperature monitored by an internal thermometer and use brine or ice water to keep the process running at approximately 24°C.

In addition to temperature control, an antifoam is also added to the process to prevent foaming, which would cause the reactor to overflow, making the process unfeasible. In a particular embodiment, antifoam is rich in polydimethylsiloxane.

Finally, the process of the present invention is also more cost effective as it performs an alcohol-free extraction, thus considerably reducing the cost with raw materials of the process.

The steps of the present invention may be modified in terms of their amounts and ratios depending on the substrate used.

In another embodiment of the present invention, the powder resulting from the process is also subjected to a micronization step, where the desired particle size can be controlled.

The following examples illustrate, but not limit, the particular embodiments of the present invention and demonstrate effectiveness of the resulting product.

### Examples

### Example 1

Babassu starch powder (1kg) was resuspended in (20L) of water. Hydrogen peroxide and sodium hydroxide were added and the temperature controlled in the range of from 20 °C to 25 °C for 2 hours. Starch was filtered and washed with acidic water until a neutral pH was set. Starch was dried to 10% moisture.

### Example 2

Bleached babassu starch obtained in example 1 was assessed for its ability to absorb oil.

To a previously weighed centrifuge tube was added a mass of high oleic microalgae oil, bleached babassu starch and an additional amount of high oleic microalgae oil. It was stirred for 1.5 minute at a speed of 2650rpm. After stirring, it was left to rest for 30 minutes and centrifuged at 3500 rpm for 30 minutes. After separating the oil (supernatant) and starch (precipitate) using a Pasteur pipette and a precision pipette, the tube was weighed with the starch and absorbed oil and the amount of absorbed oil was measured using gravimetric calculations. Analyzes were performed in quadruplicates and data and results are described in Tables 1 and 2 below.

**Table 1. Tube, starch and oil weighing data**

| | tube (g) | oil 1 (g) | starch (g) | oil 2 (g) |
|---|---|---|---|---|
| 1 | 12.95 | 5.09 | 3.01 | 10.04 |
| 2 | 13.02 | 5.02 | 3.00 | 10.01 |
| 3 | 13.08 | 5.03 | 3.06 | 10.09 |
| 4 | 12.97 | 5.11 | 3.06 | 10.01 |

**Table 2. Results of starch oil absorption**

| | tube + sample + oil (g) | sample + oil (g) | oil (g) | g oil / g sample |
|---|---|---|---|---|
| 1 | 19.12 | 6.17 | 3.17 | 1.05 |
| 2 | 19.20 | 6.18 | 3.18 | 1.06 |
| 3 | 19.43 | 6.35 | 3.29 | 1.07 |
| 4 | 19.20 | 6.23 | 3.16 | 1.03 |

The mean of the relative standard deviation of the results is 1.0556, with a relative standard deviation of 1.57%, so oil absorption capacity of bleached starch is 1.0556 gram of high oleic microalgae oil per gram of starch.

### Example 3

The following attributes of three moisturizing compositions comprising 0% (placebo), 1% and 3% bleached babassu starch prepared according to example 1 were evaluated. The chosen formulation is an oil in water emulsion of an usual body moisturizer formulation whose components are water, canola oil, glycerin, sodium acrylate copolymer, sorbitol, phenoxyethanol, benzoic acid, dehydroacetic acid, triolein, perfume, C10-30 alkyl acrylate crospolymer/acrylates, xanthan gum, tocopherol acetate, polyglyceryl-3 caprylate, disodium EDTA, sodium hydroxide, Cl 17200, Cl 15510, Cl 42090. These attributes are described in the following table 3:

**Table 3. Description of evaluated attributes**

| Attribute | Definition |
|---|---|
| Absorption point | Number of revolutions required for the product to begin to be absorbed by the skin |
| Spreadability | Easy to distribute/spread product on skin |
| Slippage | Ease to slip/slide finger over skin |
| Tackiness | Intensity with which the finger sticks to the skin |
| Immediate white residue | White film formed on the skin just after spreading the product |
| Residual white residue | White film formed on the skin 1 minute after spreading the product |
| Velvety film | "Peach skin" feel |
| Dry touch | Non-sticky, non-greasy and non-shiny skin |
| Immediate shine on skin | Intensity of light reflected on skin just after spreading the product |
| Residual shine on skin | Intensity of light reflected on skin 2 minutes after spreading the product |
| Immediate oiliness | Oily fell on the skin during and just after spreading the product |
| Residual oiliness | Oily feel on the skin 2 minutes after spreading the product |
| Immediate greasy film | Greasy feel, formation of a film on the skin just after spreading the product |
| Residual greasy film | Greasy feel, formation of a film on the skin 2 minutes after spreading the product |

Table 4 shows means, standard deviations (SD) and Fisher's Least Significant Difference (LSD) t test results for each of the assesed attributes.

**Table 4. Means, Standard Deviations and Results of Fisher's LSD Test Comparison**

| **Attribute** | **Placebo Body Moisturizer** | | **Prototype Body Moisturizer having 1% Bleached Babassu Starch** | | **Prototype Body Moisturizer having 3% Bleached Babassu Starch** | | **p-value** |
|---|---|---|---|---|---|---|---|
| | **Average Intensity (cm)** | **SD** | **Average Intensity (cm)** | **SD** | **Average Intensity (cm)** | **SD** | |
| Absorption point | 9.75 Aa | 1.16 | 9.63 Aa | 1.3 | 8.81 Bb | 0.99 | 0.0070 ** |
| Re-scaled absorption point | 0.81 Aa | 0.1 | 0.80 Aa | 0.11 | 0.73 Bb | 0.08 | 0.0070 ** |
| Spreadability | 6.73 Aa | 0.4 | 6.55 Aa | 0.49 | 6.11 Bb | 0.59 | 0.0011 ** |
| Slippage | 6.81 Aa | 0.71 | 6.63 Aa | 0.69 | 6.17 Bb | 0.76 | 0.0008 *** |
| Tackiness | 2.46 Aa | 1.05 | 1.86 Bb | 0.66 | 1.50 Bc | 0.63 | 0.0004 *** |
| Immediate white residue | 0.03 Aa | 0.01 | 0.03 Aa | 0.01 | 0.03 Aa | 0.01 | 0.303 |
| Residual white residue | 0.03 Aa | 0.01 | 0.03 Aa | 0.01 | 0.03 Aa | 0.01 | 0.3808 |
| Velvety film | 3.67 Bb | 1.11 | 3.91 ABab | 0.83 | 4.33 Aa | 0.87 | 0.0491 * |
| Dry touch | 6.66 Cc | 0.8 | 7.14 Bb | 0.9 | 7.58 Aa | 0.82 | 0.0008 *** |
| Immediate shine | 4.79 Aa | 0.57 | 4.49 Ab | 0.58 | 4.07 Bc | 0.3 | 0.0014 ** |
| Residual shine | 3.04 Aa | 0.9 | 2.96 Aa | 0.8 | 2.42 Bb | 0.78 | 0.0079 ** |
| Immediate oiliness | 4.32 Aa | 0.69 | 4.00 Bb | 0.7 | 3.66 Cc | 0.54 | 0.0004 *** |
| Residual oiliness | 2.12 Aa | 0.97 | 1.85 Bb | 0.92 | 1.57 Cc | 0.86 | 0.0001 *** |
| Immediate greasy film | 2.63 Aa | 0.85 | 2.50 Aa | 0.85 | 2.40 Aa | 0.86 | 0.1409 |
| Residual greasy film | 1.17 Aa | 0.9 | 1.06 ABa | 0.86 | 2.40 Aa | 0.76 | 0.0156 * |

| | | | | | | | |
|---|---|---|---|---|---|---|---|
| *** Significant at the 0.1% level; ** Significant at the 1% level; * Significant at the 5% level; Means followed by the same letter on each line do not differ significantly from each other at the 5% (upper case letters) or 10% (lower case letters) significance level (Fisher's LSD Test); # The Absorption Point attribute was measured in number of revolutions. | | | | | | | |

The results are listed in table 5 below, with the following intensity scores: low (0.0 - 2.0); medium-low (2.1 - 4.0); medium (4.1 - 6.0); medium-high (6.1 - 8.0); and high (8.1 - 10.0).

**Table 5. Intensity of the evaluated attributes**

| Attribute | 1% Bleached Starch Moisturizer | 3% Bleached Starch Moisturizer |
|---|---|---|
| Spreadability | Medium-high | Medium-high |
| Slippage | Medium-high | Medium-high |
| Tackiness | Low | Low |
| Immediate white residue | Low | Low |
| Residual white residue | Low | Low |
| Velvety film | Medium-low | Medium |
| Dry touch | Medium-high | Medium-high |
| Immediate shine on skin | Medium | Medium |
| Residual shine on skin | Medium-low | Medium-low |
| Immediate oiliness | Medium-low | Medium-low |
| Residual oiliness | Low | Low |
| Immediate greasy film | Medium-low | Medium-low |
| Residual greasy film | Low | Low |

Since the main benefit of bleached starch is oil absorption and accordingly to provide a sensory improvement of the formula by providing improved dry touch and less tackiness, the standard attributes were:
- dry touch
- immediate shine on skin
- residual shine on skin
- tackiness
- immediate oiliness
- residual oiliness
- immediate greasy film
- residual greasy film.

### Example 4

Table 6 below shows attributes that outperformed a placebo composition (without bleached starch) as compared to compositions having 1 and 3% bleached starch.

**Table 6. Attributes shown to be superior as compared to a placebo composition**

| Attribute | 1% Bleached Starch Moisturizer | 3% Bleached Starch Moisturizer |
|---|---|---|
| - dry touch | Superior | Superior |
| Tackiness | Superior | Superior |
| Immediate shine on skin | - | Superior |
| Residual shine on skin | - | Superior |
| Immediate oiliness | Superior | Superior |
| Residual oiliness | Superior | Superior |
| Immediate greasy film | - | - |
| Residual greasy film | - | Superior |

Reduction in tackiness, improved dry touch and reduced residual and immediate oiliness were observed in both the 1% and 3% bleached starch formulas.

In applications having 3% starch, other attributes were also noticed: faster absorption, improved formation of a velvety film, lower spreadability and slippage, lower immediate and residual shine and lower residual greasy film.

## Claims

1. A babassu bleaching process, **characterized in that** it comprises the following steps:
- sieving the substrate;
- aqueous extraction, at substrate:water ratios of from about 1:5 to about 1:20;
- adding hydrogen peroxide;
- adding sodium hydroxide at concentrations of from about 10% to about 30%;
- adding about 100 to about 200 mL of antifoam;
- filtering;
- washing with hydrochloric acid diluted in water;
- drying,
wherein the reactor temperature is maintained at about 24°C.

2. The process of claim 1, **characterized in that** the substrate is sifted using a sieve of from about 200 to about 400 mesh.

3. The process of Claim 1, **characterized in that** hydrogen peroxide has 120 volumes.

4. The process of Claim 1, **characterized in that** sodium hydroxide is added until a pH of from 11 to 12 is reached.

5. The process of Claim 1, **characterized in that** the antifoam contains polydimethylsiloxane.

6. The process of Claim 1, **characterized in that** filtration is carried out in a filter press.

7. The process of any one of claims 1 to 7, **characterized in that** babassu is from species *Orbignya phalerata.*

8. The use of babassu bleached by a process as defined in any one of claims 1 to 7, **characterized in that** it is for the preparation of a cosmetic composition.
